# EUROPÄISCHE PATENTSCHRIFT

(11) **EP 1 559 335 B1**
(45) Veröffentlichungstag und Bekanntmachung des Hinweises auf die Patenterteilung: **27.08.2008**
(21) Anmeldenummer: 05000436.5
(22) Anmeldetag: 12.01.2005
(51) Int. Cl.: A41D 13/12, A41D 27/20

(54) **Bekleidungsstück**
Clothing
Vêtement

(30) Priorität: 30.01.2004 DE 102004004852
(43) Veröffentlichungstag der Anmeldung: 03.08.2005
(73) Patentinhaber: FALKE KGaA, 57392 Schmallenberg (DE)
(72) Erfinder: Wiesenberger, Peter, 87509 Immenstadt (DE)
(74) Vertreter: Hoeger, Stellrecht & Partner Patentanwälte

(56) Entgegenhaltungen:
- EP-A- 0 447 571
- US-A- 5 375 261
- US-A- 5 935 116
- US-A1- 2002 124 295
- US-A1- 2004 009 731

## Beschreibung

Die vorliegende Erfindung betrifft ein Bekleidungsstück, insbesondere ein Shirt.

Solche Bekleidungsstücke sind bekannt.

Ebenso bekannt ist es, zur Erfassung und Überwachung von Körperfunktionen, insbesondere der Herztätigkeit, eines Menschen Körperfunktionssensoren, beispielsweise einen Herzfrequenzsensor, zu benutzen, wobei der betreffende Körperfunktionssensor mittels Riemen oder Gurten am Körper der zu überwachenden Person gehalten wird. Auf diese Weise ist eine nichtstationäre Erfassung und Überwachung beispielsweise der Herztätigkeit zur Erstellung eines Langzeit-EKGs möglich.

Bei der Befestigung solcher Körperfunktionssensoren an der zu überwachenden Person mittels Gurten und Riemen wird jedoch die Bewegungsfreiheit der zu überwachenden Person deutlich beeinträchtigt.

Die US 2004/009731 A1 offenbart ein Bekleidungsstück mit den Merkmalen des Oberbegriffs von Anspruch 1.

Der vorliegenden Erfindung liegt die Aufgabe zugrunde, eine Möglichkeit zur nichtstationären Erfassung von Körperfunktionsdaten zu schaffen, bei welcher die Bewegungsfreiheit der Person, deren Körperfunktionsdaten erfasst werden, möglichst wenig beeinträchtigt wird.

Diese Aufgabe wird erfindungsgemäß durch ein Bekleidungsstück, insbesondere ein Shirt, nach Anspruch 1 gelöst.

Ein Gehäuse eines Körperfunktionssensors kann in die Aufnahme des Bekleidungsstücks eingebracht werden, um so mindestens ein Kontaktelement des Körperfunktionssensors auf einfache und rasche Weise in Körperkontakt mit dem Träger des Bekleidungsstücks zu halten, ohne dass hierfür zusätzliche Befestigungsmittel wie Riemen oder Gurte erforderlich sind.

Das Bekleidungsstück kann, abgesehen von der Aufnahme für das Gehäuse des Körperfunktionssensors, einem herkömmlichen Bekleidungsstück entsprechen, wie es ohnehin von der Person, deren Körperfunktionsdaten erfasst werden sollen, getragen wird. Dieses Bekleidungsstück schränkt daher die Bewegungsfreiheit des Trägers nicht ein.

Das erfindungsgemäße Bekleidungsstück ermöglicht eine komfortable und flexible, dem Träger angepasste Überwachung von Körperfunktionen des Trägers insbesondere im medizinischen Bereich, vor allem bei der Überwachung von Risikopatienten, aber auch im Sportbereich, beispielsweise zur Trainingsüberwachung und -optimierung.

Das erfindungsgemäße Bekleidungsstück ist für den Träger einfach zu handhaben, anzuwenden und zu pflegen.

Um zu erreichen, dass der Körperfunktionssensor möglichst unauffällig getragen werden kann, ist die Aufnahme für das Gehäuse des Körperfunktionssensors an der Innenseite des Grundkörpers des Bekleidungsstücks angeordnet.

Dabei ist unter der Innenseite des Grundkörpers die dem Körper des Trägers zugewandte Seite zu verstehen, im Gegensatz zu der dem Körper des Trägers abgewandten Außenseite.

Besonders günstig ist es, wenn die Aufnahme so an dem Grundkörper angeordnet ist, dass sie im getragenen Zustand des Bekleidungsstücks nicht sichtbar ist.

Die Aufnahme ist als eine Aufnahmetasche ausgebildet.

Besonders günstig ist es, wenn die Aufnahmetasche ein textiles Material umfasst.

Insbesondere kann die Aufnahmetasche ein Gestrick, ein Gewirk oder ein Gewebe umfassen.

Die Aufnahmetasche ist im wesentlichen schlauchförmig ausgebildet.

Ferner ist die Aufnahmetasche vorzugsweise längs ihres Umfangs im wesentlichen nahtfrei ausgebildet, so dass sich keine störenden Nähte an dem Bekleidungsstück abzeichnen können.

Um das ganze Bekleidungsstück als eine Einheit handhaben zu können, ist es günstig, wenn die Aufnahmetasche fest mit dem Grundkörper des Bekleidungsstücks verbunden ist.

Insbesondere kann vorgesehen sein, dass die Aufnahmetasche durch Anstricken, Annähen, Kleben und/oder Verschweißen mit dem Grundkörper des Bekleidungsstücks verbunden ist.

Vorzugsweise ist vorgesehen, dass die Aufnahmetasche mit einem Kragen des Grundkörpers des Bekleidungsstücks verbunden ist. Eine im Bereich des Kragens angeordnete Aufnahmetasche ist auch im getragenen Zustand des Bekleidungsstücks für den Träger gut zugänglich.

Die Herstellung des erfindungsgemäßen Bekleidungsstücks gestaltet sich besonders einfach, wenn die Aufnahmetasche ein Material umfasst, aus dem auch der Grundkörper des Bekleidungsstücks zumindest abschnittsweise gebildet ist.

Besonders günstig ist es, wenn die Aufnahmetasche im Wesentlichen ganz aus demselben Material wie der Grundkörper des Bekleidungsstücks gebildet ist.

Um zu erreichen, dass sich die Aufnahmetasche möglichst genau an die Außenkontur des Körperfunktionssensors anpasst, ist vorzugsweise vorgesehen, dass die Aufnahmetasche ein elastisches Material, vorzugsweise Elastan, umfasst.

Ferner ist von Vorteil, wenn die Aufnahmetasche flexibel ausgebildet ist.

Um das Gehäuse des Körperfunktionssensors jederzeit in einfacher Weise aus der Aufnahme entnehmen zu können, um beispielsweise einen Speicher des Körperfunktionssensors auszulesen, neue Batterien oder Akkus in den Körperfunktionssensor einzusetzen und/oder das Bekleidungsstück zu reinigen, ist es von Vorteil, wenn die Aufnahme eine Zugangsöffnung aufweist, durch welche das Gehäuse des Körperfunktionssensors in die Aufnahme eingebracht und aus derselben entnommen werden kann.

Um zu gewährleisten, dass die Zugangsöffnung beim Einbringen bzw. Ausbringen des Gehäuses des Körperfunktionssensors so verformbar ist, dass das Gehäuse des Körperfunktionssensors die Zugangsöffnung passieren kann, weist die Zugangsöffnung vorzugsweise einen flexiblen Rand auf.

Um ein Ausreißen des Randes der Zugangsöffnung zu verhindern, ist der Rand der Zugangsöffnung vorteilhafterweise gegen eine ungewollte Erweiterung der Zugangsöffnung gesichert.

Insbesondere kann der Rand der Zugangsöffnung durch Abnähen, Verschweißen und/oder Verkleben gegen eine ungewollte Erweiterung der Zugangsöffnung gesichert sein.

Um das Gehäuse des Körperfunktionssensors auch im getragenen Zustand des Bekleidungsstücks in einfacher Weise in die Aufnahme einbringen bzw. aus der Aufnahme entnehmen zu können, hat es sich als günstig erwiesen, wenn die Zugangsöffnung in einem oberen Randbereich der Aufnahme angeordnet ist.

Um zu erreichen, dass das Gehäuse des Körperfunktionssensors innerhalb der Aufnahme eine gewünschte Arbeitsstellung einnimmt, ist es von Vorteil, wenn die Aufnahme einen Randabschnitt umfasst, der eine an einen Randabschnitt des Körperfunktionssensors angepasste Form aufweist, so dass sich der Körperfunktionssensor nach dem Einbringen in die Aufnahme in eine vorgegebene Stellung bewegt, in der sich der betreffende Randabschnitt der Aufnahme an den zugeordneten Randabschnitt des Körperfunktionssensors anschmiegt.

Insbesondere kann ein solcher Randabschnitt der Aufnahme ein geschlossenes, abgerundetes Ende der Aufnahme bilden. An diesem Ende kann die Aufnahme durch eine Naht, durch Verklebung oder ähnliches geschlossen sein.

Um zu erreichen, dass der Körperfunktionssensor in direkten Kontakt mit der Haut des Trägers gelangen kann, weist die das Gehäuse des Körperfunktionssensors aufnehmende Aufnahme vorzugsweise mindestens eine Durchtrittsöffnung für den Durchtritt eines elektrischen Kontaktelements durch eine Begrenzungswand der Aufnahme hindurch auf.

Anzahl, Position und Form der Durchtrittsöffnungen können den technischen Anforderungen des jeweils verwendeten Körperfunktionssensors angepasst gewählt werden.

Vorzugsweise ist die mindestens eine Durchtrittsöffnung in einer im getragenen Zustand des Bekleidungsstücks der Haut des Trägers des Bekleidungsstücks zugewandten Begrenzungswand der Aufnahme angeordnet.

Um diese Begrenzungswand zu verstärken und/oder ein Verrutschen des Körperfunktionssensors innerhalb der Aufnahme zu vermeiden, kann vorgesehen sein, dass die Begrenzungswand der Aufnahme, in welcher die mindestens eine Durchtrittsöffnung angeordnet ist, mit einer Beschichtung versehen ist.

Diese Beschichtung kann beispielsweise ein Laminat umfassen.

Ferner hat es sich als günstig erwiesen, wenn die Beschichtung ein Polyurethan-Material umfasst.

Die Beschichtung kann insbesondere durch Anwendung von erhöhtem Druck und/oder gegenüber der Raumtemperatur erhöhter Temperatur mit der Begrenzungswand der Aufnahme verbunden werden.

Der Grundkörper des Bekleidungsstücks umfaßt vorzugsweise ein textiles Material.

Insbesondere kann der Grundkörper ein Gestrick, ein Gewirk oder ein Gewebe umfassen.

Um unangenehme Empfindungen beim Tragen des Bekleidungsstücks, die durch Nähte ausgelöst werden, zu vermeiden, ist es von Vorteil, wenn der Grundkörper im wesentlichen nahtlos ausgebildet ist.

Um einen körpernahen Sitz des Körperfunktionssensors am Körper des Trägers und damit eine einwandfreie Funktion des Körperfunktionssensors zu gewährleisten, ist es von Vorteil, wenn der Grundkörper ein elastisches Material, vorzugsweise Elastan, umfaßt.

Insbesondere kann vorgesehen sein, daß der Grundkörper des Bekleidungsstücks unter Einsatz von Elastangarn in Verbindung mit nahtloser Strick-Technologie auf einer sogenannten Seamless-Strickmaschine hergestellt wird.

Durch die Verwendung eines Grundkörpers, der ein elastisches Material umfaßt, kann der Körperfunktionssensor gegen den Körper des Trägers vorgespannt werden.

Die Aufnahme wird so an dem Bekleidungsstück angeordnet und gestaltet, dass der Körperfunktionssensor in seiner Arbeitsstellung exakt an dem Körperteil des Trägers, dessen Funktion erfasst werden soll, positioniert ist.

Insbesondere dann, wenn Herzfunktionsdaten des Trägers erfasst werden sollen, ist es günstig, wenn die Aufnahme so an dem Bekleidungsstück angeordnet ist, dass das in der Aufnahme aufgenommene Gehäuse des Körperfunktionssensor sich im getragenen Zustand des Bekleidungsstücks im Brustbereich des Trägers, insbesondere im Bereich des Brustbeines des Trägers, befindet.

Anspruch 29 ist auf ein erfindungsgemäßes Bekleidungsstück gerichtet, das mindestens einen Körperfunktionssensor umfasst, dessen Gehäuse in der Aufnahme angeordnet ist.

Vorzugsweise ist das Gehäuse des Körperfunktionssensors zerstörungsfrei aus der Aufnahme entnehmbar, so dass das Gehäuse des Körperfunktionssensors jederzeit für Auslesezwecke, Wartungszwecke oder für die Reinigung des Bekleidungsstücks aus der Aufnahme entnommen und/oder gegen ein anderes Gehäuse eines Körperfunktionssensors ausgewechselt werden kann.

Grundsätzlich können mit dem Körperfunktionssensor beliebige Körperfunktionsdaten erfasst und ausgewertet werden.

Bei einer bevorzugten Ausgestaltung der Erfindung ist vorgesehen, dass der Körperfunktionssensor als ein Herzfrequenzsensor ausgebildet ist.

Ein geeigneter Herzfrequenzsensor, welcher in dem erfindungsgemäßen Bekleidungsstück verwendbar ist, ist beispielsweise der Herzfrequenzmesser mit der Bezeichnung "Vitaphone LS Bluetooth", der von der Firma Vitaphone GmbH Telemedizin, O 7, 18 in 68161 Mannheim, Deutschland, vertrieben wird.

Um den Körperfunktionssensor in einfacher Weise und ohne Zwischenschaltung zusätzlicher Kabel etc. in direkten Kontakt mit der Haut des Trägers des Bekleidungsstücks bringen zu können, umfaßt der Körperfunktionssensor vorteilhafterweise mindestens ein elektrisches Kontaktelement, das sich im getragenen Zustand des Bekleidungsstücks durch eine Durchtrittsöffnung der Aufnahme hindurch erstreckt.

Ferner umfaßt der Körperfunktionssensor vorzugsweise mindestens ein Körperkontaktelement, das sich im getragenen Zustand des Bekleidungsstücks in direktem Kontakt mit dem Körper, insbesondere mit der Haut, des Trägers des Bekleidungsstücks befindet.

Der Körperfunktionssensor kann eine Einrichtung zum Speichern von Körperfunktionsdaten und/oder eine Einrichtung zum drahtlosen Übertragen von Körperfunktionsdaten zu einem Körperfunktionsdaten-Empfänger umfassen.

Ferner kann vorgesehen sein, daß der Körperfunktionssensor mit einem GPS-Modul versehen ist, welches es ermöglicht, den Aufenthaltsort des Trägers des Bekleidungsstücks jederzeit genau zu bestimmen, damit im Falle einer Krisensituation, welche aus den vom Körperfunktionssensor an den Körperfunktionsdaten-Empfänger übertragenen Körperfunktionsdaten erkennbar ist, der Träger des Bekleidungsstücks geortet und mit gegebenenfalls erforderlichen Hilfeleistungen versorgt werden kann.

Bei einer bevorzugten Ausgestaltung des erfindungsgemäßen Bekleidungsstücks ist vorgesehen, daß das Bekleidungsstück einen im getragenen Zustand des Bekleidungsstücks die Brust des Trägers überdeckenden Brustbereich umfaßt.

Das erfindungsgemäße Bekleidungsstück kann mit oder ohne Armteile ausgebildet sein.

Bei einer bevorzugten Ausgestaltung der Erfindung ist vorgesehen, daß das Bekleidungsstück Armteile umfaßt.

Das erfindungsgemäße Bekleidungsstück ist vorzugsweise als Wäscheteil gearbeitet, welches direkt auf dem Körper des Trägers getragen wird.

Weitere Merkmale und Vorteile der Erfindung sind Gegenstand der nachfolgenden Beschreibung und der zeichnerischen Darstellung eines Ausführungsbeispiels.

In den Zeichnungen zeigen:
- Fig. 1: eine schematische Vorderansicht eines Bekleidungsstücks in Form eines Shirts, das an seiner Innenseite mit einer Aufnahmetasche für einen Herzfrequenzsensor versehen ist, im getragenen Zustand;
- Fig. 2: eine teilweise geschnittene Seitenansicht des Bekleidungsstücks aus Fig. 1, mit der Blickrichtung in Richtung des Pfeils 2 in Fig. 1;
- Fig. 3: einen schematischen Längsschnitt durch das Bekleidungsstück mit der Aufnahmetasche für den Herzfrequenzsensor und durch den darin aufgenommenen Herzfrequenzsensor sowie die Haut des Trägers;
- Fig. 4: eine schematische Draufsicht auf die Aufnahmetasche des Bekleidungsstücks, von der dem Träger des Bekleidungsstücks zugewandten Seite der Aufnahmetasche aus gesehen; und
- Fig. 5: eine schematische perspektivische Darstellung eines Herzfrequenzsensors, dessen Gehäuse in die Aufnahmetasche des Bekleidungsstücks eingebracht werden kann.

Gleiche oder funktional äquivalente Elemente sind in allen Figuren mit denselben Bezugszeichen bezeichnet.

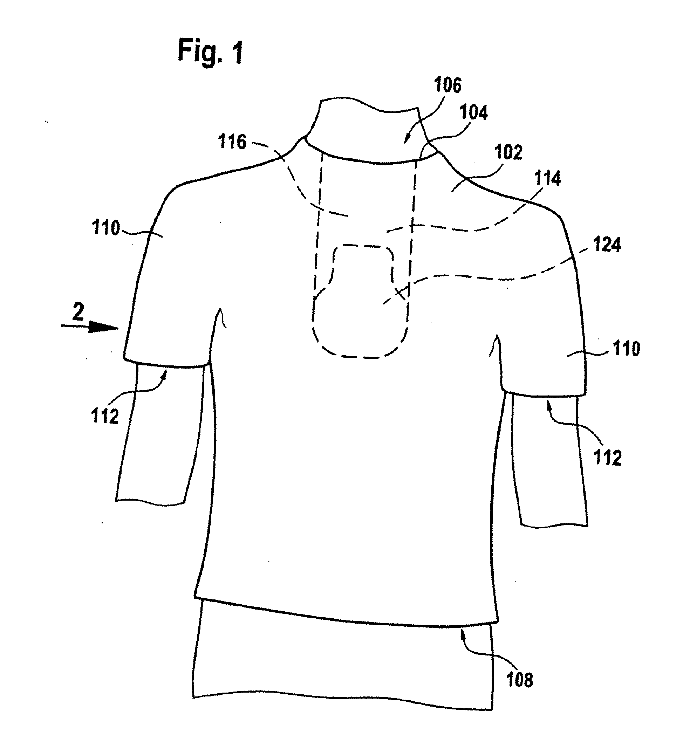

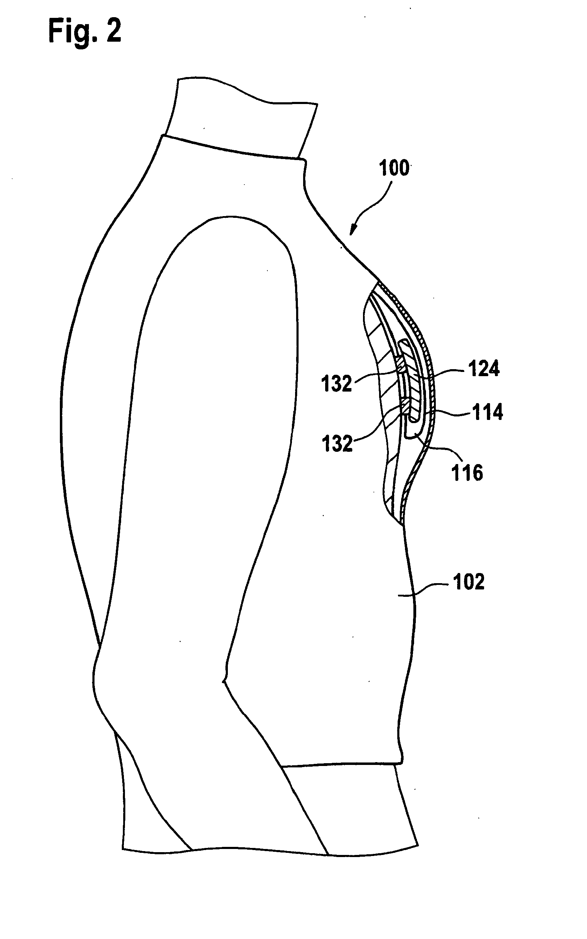

Ein in den Fig. 1 bis 4 dargestelltes, als Ganzes mit 100 bezeichnetes Bekleidungsstück ist beispielsweise als ein Kurzarm-Shirt ausgebildet und umfasst einen Grundkörper 102, der an seinem oberen Rand mit einem Kragen 104 versehen ist, welcher eine Halsdurchtrittsöffnung 106 des Bekleidungsstücks 100 umgibt. Ferner weist der Grundkörper 102 des Bekleidungsstücks 100 an seinem unteren Rand eine Rumpfdurchtrittsöffnung 108 sowie in seinem oberen Bereich zwei Armteile 110 auf, die an Armdurchtrittsöffnungen 112 enden.

Der Rumpfteil des Grundkörpers 102 des Bekleidungsstücks 100 ist vorzugsweise nahtfrei ausgebildet.

Der Grundkörper 102 ist als ein Gestrick ausgebildet, das aus einem beliebigen Textilfasermaterial, insbesondere aus einem Naturfasergarn, aus einem Kunstfasergarn und/oder aus einem Mischgarn gebildet sein kann.

Vorzugsweise umfaßt das Gestrick des Grundkörpers 102 ein elastisches Garn, insbesondere Elastan, wodurch ein straffes Anliegen des Grundkörpers 102 am Körper des Trägers und somit ein körpernaher Sitz des Bekleidungsstücks 100 erzielt wird.

Insbesondere kann vorgesehen sein, daß der Grundkörper 102 aus einem Umwindungsgarn gestrickt ist, welches einen zentralen Faden aus Elastan umfaßt, der mit einem Faden aus Polyamid wendelförmig umwunden ist.

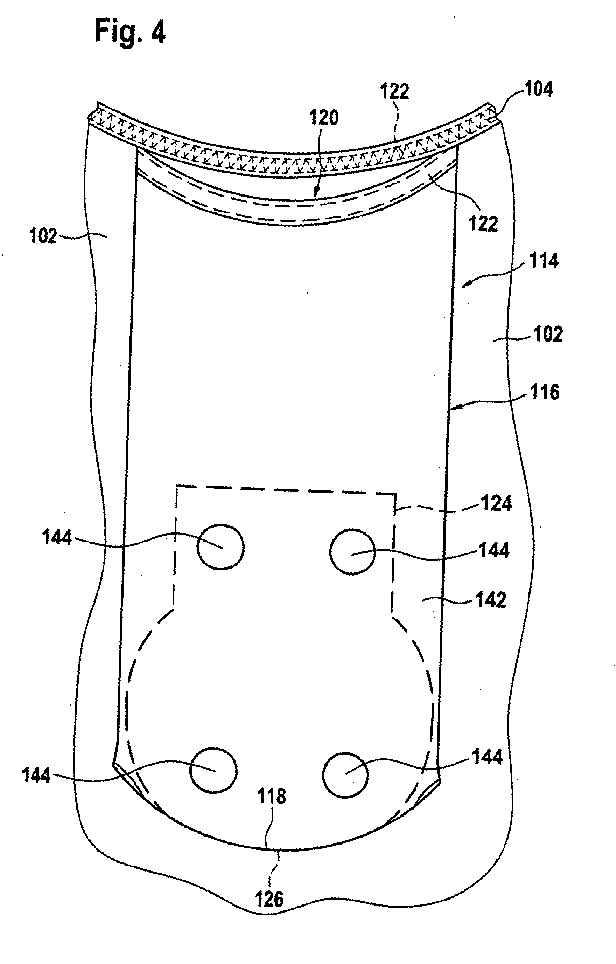

Wie am besten aus Fig. 4 zu ersehen ist, ist das Bekleidungsstück 100 mit einer Aufnahme 114 in Form einer flexiblen Aufnahmetasche 116 versehen.

Die Aufnahmetasche 116 ist als ein Schlauch ausgebildet, der an seinem unteren Ende durch einen kreisabschnittsförmig ausgebildeten Randbereich 118 geschlossen ist.

An ihrem oberen Ende ist die Aufnahmetasche 116 mit einer Zugangsöffnung 120 versehen, welche von einem flexiblen Rand 122 ringförmig umgeben ist.

Um ein Ausreißen des Randes 122 der Zugangsöffnung 120 zu verhindern, ist der Rand 122 umgeschlagen und laufmaschensicher abgenäht, verschweißt und/oder verklebt.

Ferner ist die (in der Blickrichtung der Fig. 4 gesehen) hintere Hälfte des Randes 122 an den Kragen 104 des Bekleidungsstücks 100 angenäht, um die Aufnahmetasche 116 an dem Grundkörper 102 des Bekleidungsstücks 100 zu befestigen.

Diese Befestigung kann alternativ oder ergänzend zu einem Vernähen auch durch ein Verkleben und/oder Verschweißen erfolgen.

Die Aufnahmetasche 116 ist auf der im getragenen Zustand des Bekleidungsstücks 100 dem Träger zugewandten Innenseite des Bekleidungsstücks 100 angeordnet.

Auch die Aufnahmetasche 116 kann aus einem beliebigen textilen Material gebildet sein.

Insbesondere kann vorgesehen sein, dass die Aufnahmetasche 116 aus einem Naturfasergarn, einem Kunstfasergarn und/oder einem Mischgarn gestrickt ist.

Vorzugsweise ist vorgesehen, dass die Aufnahmetasche 116 ein elastisches Material, insbesondere Elastan, umfasst.

Besonders günstig ist es, wenn die Aufnahmetasche 116 aus demselben Material gebildet ist wie der Grundkörper 102 des Bekleidungsstücks.

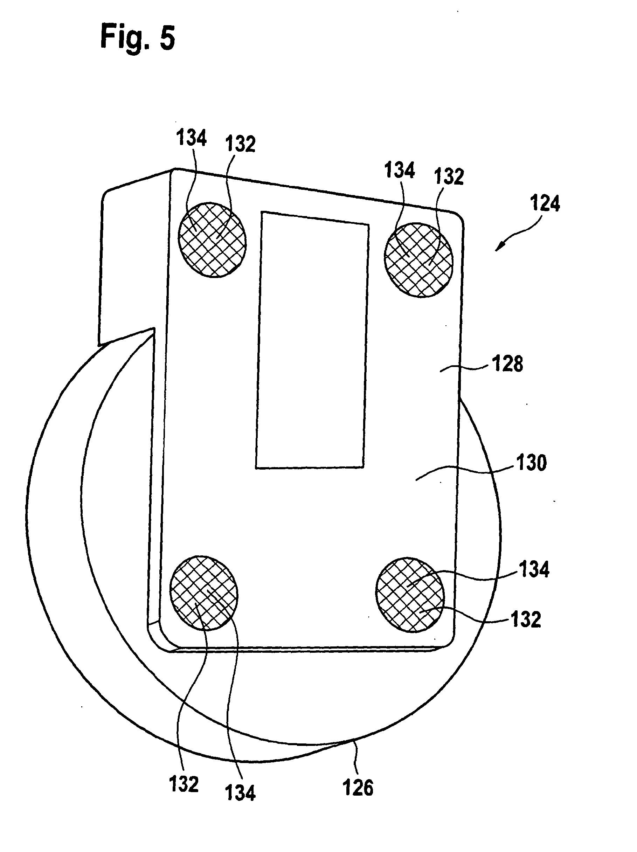

Durch die Zugangsöffnung 120 kann das Gehäuse 128 des in Fig. 5 beispielhaft dargestellten Herzfrequenzsensors 124 in die Aufnahmetasche 116 eingebracht und bei Bedarf, insbesondere zum Zwecke der Wartung des Herzfrequenzsensors 124 und/oder für eine Reinigung des Bekleidungsstücks 100, wieder aus der Aufnahmetasche 116 entnommen werden.

Aufgrund der Schwerkraft rutscht das Gehäuse 128 des Herzfrequenzsensors 124 in der Aufnahmetasche 116 nach unten, bis es mit seinem unteren Randbereich 126 an dem unteren Randbereich 118 der Aufnahmetasche 116 anliegt. Das in dieser Arbeitsstellung befindliche Gehäuse 128 des Herzfrequenzsensors 124 ist in Fig. 4 durch die gebrochenen Linien angedeutet.

Die Länge der Aufnahmetasche 116 ist so bemessen, dass der Herzfrequenzsensor 124 in seiner Arbeitsstellung exakt in Höhe des Brustbeines des Trägers positioniert ist.

Um eine möglichst genaue Positionierung des Herzfrequenzsensors 124 in der Arbeitsstellung zu ermöglichen, ist die Form des unteren Randbereichs 118 der Aufnahmetasche 116 an die Form des unteren Randbereichs 126 des Herzfrequenzsensors 124 angepasst.

Wie am besten aus Fig. 5 zu ersehen ist, umfasst der Herzfrequenzsensor 124 ein Gehäuse 128 mit einer im wesentlichen ebenen Anlagefläche 130, über welche mehrere, beispielsweise vier, Körperkontaktelemente 132 in Form von im wesentlichen scheibenförmigen Kontaktplatten 134 aus einem elektrisch leitfähigen Material überstehen.

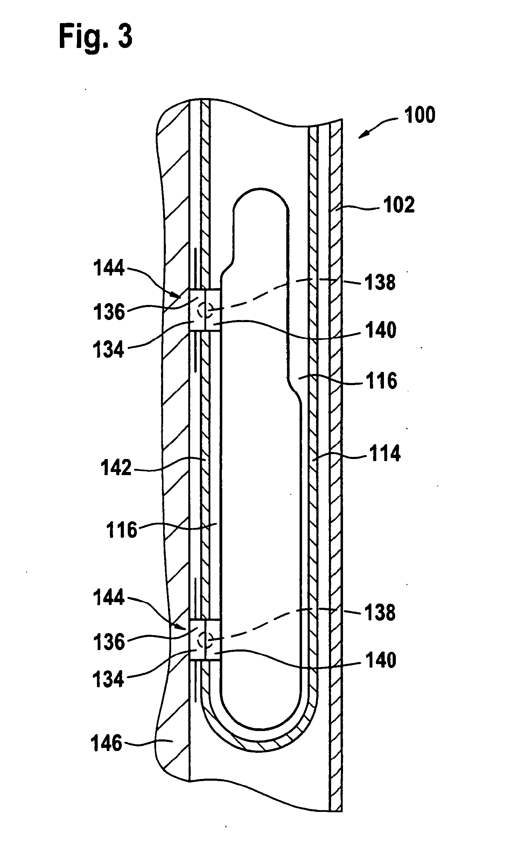

Wie am besten aus Fig. 3 zu ersehen ist, ist die Rückseite jeder der Kontaktplatten 134 mit einer Aufnahme versehen, welche dem Federteil 136 eines Druckknopfes entspricht.

Im Betriebszustand des Herzfrequenzsensors 124 ist jede der Kontaktplatten 134 mit diesem Federteil 136 auf ein hierzu komplementär ausgebildetes Kugelteil 138 aufgesetzt, welches aus dem Gehäuse 128 des Herzfrequenzsensors 124 herausragt, und mit diesem Kugelteil 138 verrastet, so dass jede der Kontaktplatten 134 lösbar an dem jeweiligen Kugelteil 138 gehalten ist.

Die Kugelteile 138 sind ebenfalls aus einem elektrisch leitfähigen Material gebildet, so daß elektrische Signale von den Kontaktplatten 134 über die Kugelteile 138 in das Innere des Gehäuses 128 des Herzfrequenzsensors 124 übertragen werden können.

Die Kugelteile 138 bilden somit elektrisch leitende Kontaktelemente 140, welche die Kontaktplatten 134 mit einer elektrischen Schaltung in dem Gehäuse 128 des Herzfrequenzsensors 124 verbinden.

Um den Durchtritt dieser elektrischen Kontaktelemente 140 durch die dem Träger des Bekleidungsstücks 100 zugewandte flexible Begrenzungswand 142 der Aufnahmetasche 116 zu ermöglichen, ist diese Begrenzungswand 142 mit mehreren Durchtrittsöffnungen 144 versehen, deren Anzahl der Anzahl der Kontaktelemente 140 des Herzfrequenzsensors 124 und deren Position an der Aufnahmetasche 116 der Position der Kontaktelemente 140 in der Arbeitsstellung des Herzfrequenzsensors 124 entspricht (siehe Fig. 3 und Fig. 4).

Die Durchtrittsöffnungen 144 sind beispielsweise im wesentlichen kreisförmig ausgebildet. Ihr Durchmesser ist größer als der Durchmesser der Kontaktelemente 140, damit die Kontaktelemente 140 durch die Durchtrittsöffnungen 144 hindurchtreten können. Der Durchmesser der Durchtrittsöffnungen 144 ist kleiner als der Durchmesser der Kontaktplatten 134, um zu verhindern, daß die Kontaktplatten 134 durch die Durchtrittsöffnungen 144 in den Innenraum der Aufnahmetasche 116 gelangen und sich so von der Haut 146 des Trägers entfernen.

Die Kontaktelemente 140 könnten auch an ihrem körperseitigen Ende eben ausgebildet sein und direkt an der Haut des Trägers anliegen.

Um ein Ausreißen der Ränder der Durchtrittsöffnungen 144 zu vermeiden, ist vorgesehen, daß die dem Innenraum der Aufnahmetasche 116 zugewandte Seite der Begrenzungswand 142 mit einer verstärkenden Beschichtung versehen ist.

Diese Beschichtung kann insbesondere als ein Laminat, beispielsweise aus Polyurethan, ausgebildet sein, welches mit der Innenseite der Begrenzungswand 142 verklebt wird.

Die Verbindung zwischen dem Laminat und dem Material der Aufnahmetasche 116 erfolgt unter erhöhtem Druck und bei einer erhöhten Temperatur im Bereich von ungefähr 160° C.

Die Beschichtung ist flexibel ausgebildet, um die Verformbarkeit der Aufnahmetasche 116 und damit die Anpassung der Form der Aufnahmetasche 116 an die Form des Herzfrequenzsensors 124 nicht zu beeinträchtigen.

Vorzugsweise ist die Beschichtung dünn ausgebildet. Insbesondere kann vorgesehen sein, daß die Dicke der Beschichtung weniger als ungefähr 60 µm, vorzugsweise weniger als ungefähr 30 µm, beträgt.

Die Beschichtung an der Innenseite der Begrenzungswand 142 hat die zusätzliche Wirkung, daß ein Verrutschen des Herzfrequenzsensors 124 aus der Arbeitsstellung innerhalb der Aufnahmetasche 116 vermieden wird.

Bei der Herstellung des Bekleidungsstücks 100 wird vorzugsweise so vorgegangen, daß zunächst die Begrenzungswand 142 an ihrer Innenseite mit der Beschichtung versehen wird und anschließend die Durchtrittsöffnungen 144 durch Ausstanzen erzeugt werden.

Die der Innenseite der Begrenzungswand 142 gegenüberliegende Innenseite der Aufnahmetasche 116 bleibt vorzugsweise unbeschichtet.

Um den zunächst außerhalb der Aufnahmetasche 116 befindlichen Herzfrequenzsensor 124 betriebsbereit zu machen, werden die Kontaktplatten 134 von den Kontaktelementen 140 abgenommen und auf die Haut 146 des Trägers geklebt (siehe Fig. 3).

Das Gehäuse 128 des Herzfrequenzsensors 124 wird durch die Zugangsöffnung 120 in die Aufnahmetasche 116 eingeführt und bewegt sich nach unten, bis es die Arbeitsstellung erreicht, in welcher die Kontaktelemente 140 des Herzfrequenzsensors 124 im Bereich der jeweils zugeordneten Durchtrittsöffnung 144 angeordnet sind und durch die jeweils zugeordnete Durchtrittsöffnung 144 aus der Aufnahmetasche 116 herausragen. Falls eines oder mehrere der Kontaktelemente 140 noch nicht durch die jeweils zugeordnete Durchtrittsöffnung 144 herausragen, kann die flexible Begrenzungswand 142 der Aufnahmetasche 116 relativ zu dem Gehäuse 128 des Herzfrequenzsensors 124 so bewegt werden, dass die Kontaktelemente 140 aus der jeweils zugeordneten Durchtrittsöffnung 144 herausragen.

Anschließend wird das Bekleidungsstück 100 vom Träger angelegt, wobei die Kontaktelemente 140 im Bereich der auf die Haut 146 des Trägers aufgeklebten Kontaktplatten 134 zu liegen kommen und durch Druck des Trägers auf das Bekleidungsstück 100 von außen im Bereich des Herzfrequenzsensors 124 mit den Kontaktplatten 134 verrastet werden können.

Wenn der Grundkörper 102 des Bekleidungsstücks 100 ein elastisches Material umfasst, so übt der Grundkörper 102 ferner eine elastische Vorspannung auf den Herzfrequenzsensor 124 aus, welche so gerichtet ist, dass sie den Herzfrequenzsensor 124 gegen die Haut 146 des Trägers des Bekleidungsstückes 100 drückt.

Nach Herstellung der Verbindung zwischen den Kontaktplatten 134 und den Kontaktelementen 140 ist der Herzfrequenzsensor 124 betriebsbereit.

Die über die Kontaktplatten 134 vom Herzfrequenzsensor 124 erhaltenen Signale werden von einer elektronischen Schaltung im Inneren des Gehäuses 128 ausgewertet, und die so erhaltenen Daten werden in einem Speicher innerhalb des Gehäuses 128, der nach Entnahme des Gehäuses 128 des Herzfrequenzsensors 124 aus der Aufnahmetasche 116 ausgelesen wird, abgelegt und/oder mittels einer drahtlosen Übertragungstechnik, beispielsweise mittels Bluetooth-, WLAN-, UMTS- oder Mobilfunktechnik, zu einem von dem Träger des Bekleidungsstücks 100 entfernten Empfänger übertragen und dort abgespeichert und/oder ausgewertet.

Außerdem kann der Herzfrequenzsensor 124 ein GPS-Modul umfassen, durch welches der Träger des Bekleidungsstücks ortbar ist.

Der Herzfrequenzsensor 124 kann ferner mit einem Warntongeber versehen sein, welcher in Abhängigkeit von den vom Herzfrequenzsensor ermittelten Daten, beispielsweise bei Überschreiten einer bestimmten vorgegebenen Herzfrequenz, einen Warnton erzeugt.

Der Herzfrequenzsensor 124 wird mittels Batterien oder Akkus, die in dem Gehäuse 128 untergebracht sind, betrieben.

Das Gehäuse 128 des Herzfrequenzsensors 124 weist beispielsweise eine Höhe von ungefähr 100 mm, eine Breite von ungefähr 80 mm und eine Tiefe von ungefähr 15 mm auf.

Wenn das Gehäuse 128 des Herzfrequenzsensors 124 aus der Aufnahmetasche 116 entnommen wird, kann das Bekleidungsstück 100 in gewöhnlicher Weise durch Waschen gereinigt werden.

Ferner ist das Bekleidungsstück 100 ohne jede Einschränkung auch ohne den Herzfrequenzsensor 124 benutzbar.

Das Bekleidungsstück 100 besitzt somit keinerlei Einschränkungen in seinen Trage- und Pflegeeigenschaften.

Die Aufnahme 114 des Gehäuses 128 des Herzfrequenzsensors 124 ist von außen nicht erkennbar. Allenfalls ist bei straff am Körper des Trägers anliegendem Grundkörper 102 im Brustbereich des Bekleidungsstücks 100 eine von dem Gehäuse 128 des Herzfrequenzsensors 124 hervorgerufene Auswölbung wahrnehmbar (siehe Fig. 1).

## Patentansprüche

1. Bekleidungsstück, insbesondere Shirt, umfassend einen Grundkörper (102) und eine an dem Grundkörper (102) angeordnete Aufnahme (114) für ein Gehäuse (128) eines Körperfunktionssensors (124)wobei die Aufnahme (114) als eine Aufnahmetasche (116) ausgebildet ist,
**dadurch gekennzeichnet,**
**dass** die Aufnahme (114) an der Innenseite des Grundkörpers (102) angeordnet ist und
**dass** die Aufnahmetasche (116) im wesentlichen schlauchförmig ausgebildet ist.

2. Bekleidungsstück nach Anspruch 1, **dadurch gekennzeichnet, dass** die Aufnahme (114) so an dem Grundkörper (102) angeordnet ist, dass sie im getragenen Zustand des Bekleidungsstücks (100) nicht sichtbar ist.

3. Bekleidungsstück nach einem der Ansprüche 1 oder 2, **dadurch gekennzeichnet, dass** die Aufnahmetasche (116) ein textiles Material umfasst.

4. Bekleidungsstück nach einem der Ansprüche 1 bis 3, **dadurch gekennzeichnet, dass** die Aufnahmetasche (116) ein Gestrick oder Gewirk umfasst.

5. Bekleidungsstück nach einem der Ansprüche 1 bis 4, **dadurch gekennzeichnet, dass** die Aufnahmetasche (116) im wesentlichen nahtfrei aus-gebildet ist.

6. Bekleidungsstück nach einem der Ansprüche 1 bis 5, **dadurch gekennzeichnet, dass** die Aufnahmetasche (116) fest mit dem Grundkörper (102) des Bekleidungsstücks (100) verbunden ist.

7. Bekleidungsstück nach Anspruch 6, **dadurch gekennzeichnet, dass** die Aufnahmetasche (116) durch Anstricken, Annähen, Kleben und/oder Verschweißen mit dem Grundkörper (102) des Bekleidungsstücks (100) verbunden ist.

8. Bekleidungsstück nach einem der Ansprüche 6 oder 7, **dadurch gekennzeichnet, dass** die Aufnahmetasche (116) mit einem Kragen (104) des Grundkörpers (102) des Bekleidungsstücks (100) verbunden ist.

9. Bekleidungsstück nach einem der Ansprüche 1 bis 8, **dadurch gekennzeichnet, dass** die Aufnahmetasche (116) ein Material umfasst, aus dem auch der Grundkörper (102) des Bekleidungsstücks (100) zumindest abschnittsweise gebildet ist.

10. Bekleidungsstück nach einem der Ansprüche 1 bis 9, **dadurch gekennzeichnet, dass** die Aufnahmetasche (116) ein elastisches Material, vorzugsweise Elastan, umfasst.

11. Bekleidungsstück nach einem der Ansprüche 1 bis 10, **dadurch gekennzeichnet, dass** die Aufnahmetasche (116) flexibel ausgebildet ist.

12. Bekleidungsstück nach einem der Ansprüche 1 bis 11, **dadurch gekennzeichnet, dass** die Aufnahme (114) eine Zugangsöffnung (120) aufweist, durch welche das Gehäuse (128) des Körperfunktionssensörs (124) in die Aufnahme (114) eingebracht und aus derselben entnommen werden kann.

13. Bekleidungsstück nach Anspruch 12, **dadurch gekennzeichnet, dass** die Zugangsöffnung (120) einen flexiblen Rand (122) aufweist.

14. Bekleidungsstück nach Anspruch 13, **dadurch gekennzeichnet, dass** die Zugangsöffnung (120) einen gegen eine ungewollte Erweiterung der Zugangsöffnung (120) gesicherten Rand (122) aufweist.

15. Bekleidungsstück nach Anspruch 14, **dadurch gekennzeichnet, dass** der Rand (122) der Zugangsöffnung (120) durch Abnähen, Verschweißen und/oder Verkleben gegen eine ungewollte Erweiterung der Zugangsöffnung (120) gesichert ist.

16. Bekleidungsstück nach einem der Ansprüche 12 bis 15, **dadurch gekennzeichnet, dass** die Zugangsöffnung (120) in einem oberen Randbereich der Aufnahme (114) angeordnet ist.

17. Bekleidungsstück nach einem der Ansprüche 1 bis 16, **dadurch gekennzeichnet, dass** die Aufnahme (114) einen Randabschnitt (118) umfasst, der eine an einen Randabschnitt (126) des Körperfunktionssensors (124) angepasste Form aufweist.

18. Bekleidungsstück nach einem der Ansprüche 1 bis 17, **dadurch gekennzeichnet, dass** die Aufnahme (114) mindestens eine Durchtrittsöffnung (144) für den Durchtritt eines elektrischen Kontaktelements (140) durch eine Begrenzungswand (142) der Aufnahme (114) aufweist.

19. Bekleidungsstück nach Anspruch 18, **dadurch gekennzeichnet, dass** die mindestens eine Durchtrittsöffnung (144) in einer im getragenen Zustand des Bekleidungsstücks (100) der Haut (146) des Trägers des Bekleidungsstücks (100) zugewandten Begrenzungswand (142) der Aufnahme (114) angeordnet ist.

20. Bekleidungsstück nach einem der Ansprüche 18 oder 19, **dadurch gekennzeichnet, dass** die Begrenzungswand (142) der Aufnahme (114), in welcher die mindestens eine Durchtrittsöffnung (144) angeordnet ist, mit einer Beschichtung versehen ist.

21. Bekleidungsstück nach Anspruch 20, **dadurch gekennzeichnet, dass** die Beschichtung ein Laminat umfasst.

22. Bekleidungsstück nach einem der Ansprüche 20 oder 21, **dadurch gekennzeichnet, dass** die Beschichtung ein Polyurethan-Material umfasst.

23. Bekleidungsstück nach einem der Ansprüche 20 bis 22, **dadurch gekennzeichnet, dass** die Beschichtung durch Anwendung von erhöhtem Druck und/oder erhöhter Temperatur mit der Begrenzungswand (142) der Aufnahme (114) verbunden worden ist.

24. Bekleidungsstück nach einem der Ansprüche 1 bis 23, **dadurch gekennzeichnet, dass** der Grundkörper (102) ein textiles Material umfasst.

25. Bekleidungsstück nach einem der Ansprüche 1 bis 24, **dadurch gekennzeichnet, dass** der Grundkörper (102) ein Gestrick oder Gewirk umfasst.

26. Bekleidungsstück nach einem der Ansprüche 1 bis 25, **dadurch gekennzeichnet, dass** der Grundkörper (102) im wesentlichen nahtlos ausgebildet ist.

27. Bekleidungsstück nach einem der Ansprüche 1 bis 26, **dadurch gekennzeichnet, dass** der Grundkörper (102) ein elastisches Material, vorzugsweise Elastan, umfasst.

28. Bekleidungsstück nach einem der Ansprüche 1 bis 27, **dadurch gekennzeichnet, dass** die Aufnahme (114) so an dem Bekleidungsstück (100) angeordnet ist, dass das in der Aufnahme (114) aufgenommene Gehäuse (128) des Körperfunktionssensors (124) sich im getragenen Zustand des Bekleidungsstücks (100) im Brustbereich des Trägers befindet.

29. Bekleidungsstück nach einem der Ansprüche 1 bis 28, **dadurch gekennzeichnet, dass** das Bekleidungsstück (100) mindestens einen Körperfunktionssensor (124) umfasst, dessen Gehäuse (128) in der Aufnahme (114) angeordnet ist.

30. Bekleidungsstück nach Anspruch 29, **dadurch gekennzeichnet, dass** das Gehäuse (128) des Körperfunktionssensors (124) zerstörungsfrei aus der Aufnahme (114) entnehmbar ist.

31. Bekleidungsstück nach einem der Ansprüche 29 oder 30, **dadurch gekennzeichnet, dass** der Körperfunktionssensor (124) als ein Herzfrequenzsensor ausgebildet ist.

32. Bekleidungsstück nach einem der Ansprüche 29 bis 31, **dadurch gekennzeichnet, dass** der Körperfunktionssensor (124) mindestens ein elektrisches Kontaktelement (140) umfasst, das sich im getragenen Zustand des Bekleidungsstücks (100) durch eine Durchtrittsöffnung (144) der Aufnahme (114) hindurch erstreckt.

33. Bekleidungsstück nach einem der Ansprüche 29 bis 32, **dadurch gekennzeichnet, dass** der Körperfunktionssensor (124) mindestens ein Körperkontaktelement (134) umfasst, das sich im getragenen Zustand des Bekleidungsstücks (100) in direktem Kontakt mit dem Körper, insbesondere mit der Haut (146), des Trägers des Bekleidungsstücks (100) befindet.

34. Bekleidungsstück nach einem der Ansprüche 29 bis 33, **dadurch gekennzeichnet, dass** der Körperfunktionssensor (124) eine Einrichtung zum Speichern von Körperfunktionsdaten und/oder eine Einrichtung zum drahtlosen Übertragen von Körperfunktionsdaten zu einem Körperfunktionsdaten-Empfänger umfasst.

35. Bekleidungsstück nach einem der Ansprüche 1 bis 34, **dadurch gekennzeichnet, dass** das Bekleidungsstück (100) einen im getragenen Zustand des Bekleidungsstücks (100) die Brust des Trägers überdeckenden Brustbereich umfasst.

36. Bekleidungsstück nach einem der Ansprüche 1 bis 35, **dadurch gekennzeichnet, dass** das Bekleidungsstück (100) Armteile (110) umfasst.

## Claims

1. Item of clothing, in particular a shirt, comprising a basic body (102) and a receptacle (114), which is disposed on the basic body (102), for a housing (128) of a body function sensor (124), wherein the receptacle (114) is formed as a receiving pocket (116),
**characterised in that**
the receptacle (114) is disposed on the inner side of the basic body (102) and
the receiving pocket (116) is substantially tubular in formation.

2. Item of clothing as claimed in claim 1, **characterised in that** the receptacle (114) is disposed on the basic body (102) in such a manner that it is not visible when the item of clothing (100) is being worn.

3. Item of clothing as claimed in any one of claims 1 or 2, **characterised in that** the receiving pocket (116) comprises a textile material.

4. Item of clothing as claimed in any one of claims 1 to 3, **characterised in that** the receiving pocket (116) comprises a knitted fabric or interlaced yarns.

5. Item of clothing as claimed in any one of claims 1 to 4, **characterised in that** the receiving pocket (116) is formed substantially without any seams.

6. Item of clothing as claimed in any of claims 1 to 5, **characterised in that** the receiving pocket (116) is fixedly connected to the basic body (102) of the item of clothing (100).

7. Item of clothing as claimed in claim 6, **characterised in that** the receiving pocket (116) is connected to the basic body (102) of the item of clothing (100) by knitting, stitching, gluing and/or welding.

8. Item of clothing as claimed in any one of claims 6 or 7, **characterised in that** the receiving pocket (116) is connected to a collar (104) of the basic body (102) of the item of clothing (100).

9. Item of clothing as claimed in any one of claims 1 to 8, **characterised in that** the receiving pocket (116) comprises a material, from which at least portions of the basic body (102) of the item of clothing (100) are also formed.

10. Item of clothing as claimed in any one of claims 1 to 9, **characterised in that** the receiving pocket (116) comprises an elastic material, preferably elastane.

11. Item of clothing as claimed in any one of claims 1 to 10, **characterised in that** the receiving pocket (116) is formed in a flexible manner.

12. Item of clothing as claimed in any one of claims 1 to 11, **characterised in that** the receptacle (114) comprises an access opening (120), through which the housing (128) of the body function sensor (124) can be introduced into and removed from the receptacle (114).

13. Item of clothing as claimed in claim 12, **characterised in that** the access opening (120) comprises a flexible edge (122).

14. Item of clothing as claimed in claim 13, **characterised in that** the access opening (120) comprises an edge (122) which is protected against any undesired widening of the access opening (120).

15. Item of clothing as claimed in claim 14, **characterised in that** the edge (122) of the access opening (120) is protected against any undesired widening of the access opening (120) by top-stitching, welding and/or gluing.

16. Item of clothing as claimed in any one of claims 12 to 15, **characterised in that** the access opening (120) is disposed in an upper edge region of the receptacle (114).

17. Item of clothing as claimed in any one of claims 1 to 16, **characterised in that** the receptacle (114) comprises an edge portion (118) which has a shape which is adapted to the edge portion (126) of the body function sensor (124).

18. Item of clothing as claimed in any one of claims 1 to 17, **characterised in that** the receptacle (114) comprises at least one opening (144) to allow the passage of an electrical contact element (140) through a boundary wall (142) of the receptacle (114).

19. Item of clothing as claimed in claim 18, **characterised in that** the at least one opening (144) is disposed in a boundary wall (142) of the receptacle (114) which, when the item of clothing (100) is being worn, faces towards the skin (146) of the wearer of the item of clothing (100).

20. Item of clothing as claimed in any one of claims 18 or 19, **characterised in that** the boundary wall (142) of the receptacle (114), in which the at least one opening (144) is disposed, is provided with a coating.

21. Item of clothing as claimed in claim 20, **characterised in that** the coating comprises a laminate.

22. Item of clothing as claimed in any one of claims 20 or 21, **characterised in that** the coating comprises a polyurethane material.

23. Item of clothing as claimed in any one of claims 20 to 22, **characterised in that** the coating has been connected to the boundary wall (142) of the receptacle (114) by the application of increased pressure and/or elevated temperature.

24. Item of clothing as claimed in any one of claims 1 to 23, **characterised in that** the basic body (102) comprises a textile material.

25. Item of clothing as claimed in any one of claims 1 to 24, **characterised in that** the basic body (102) comprises a knitted fabric or interlaced yarns.

26. Item of clothing as claimed in any one of claims 1 to 25, **characterised in that** the basic body (102) is formed substantially without any seams.

27. Item of clothing as claimed in any one of claims 1 to 26, **characterised in that** the basic body (102) comprises an elastic material, preferably elastane.

28. Item of clothing as claimed in any one of claims 1 to 27, **characterised in that** the receptacle (114) is disposed on the item of clothing (100) in such a manner that, when the item of clothing (100) is being worn, the housing (128) of the body function sensor (124) received in the receptacle (114) is located in the wearer's chest region.

29. Item of clothing as claimed in any one of claims 1 to 28, **characterised in that** the item of clothing (100) comprises at least one body function sensor (124), the housing (128) of which is disposed in the receptacle (114).

30. Item of clothing as claimed in claim 29, **characterised in that** the housing (128) of the body function sensor (124) can be non-destructively removed from the receptacle (114).

31. Item of clothing as claimed in any one of claims 29 or 30, **characterised in that** the body function sensor (124) is formed as a heart rate sensor.

32. Item of clothing as claimed in any one of claims 29 to 31, **characterised in that** the body function sensor (124) comprises at least one electrical contact element (140) which, when the item of clothing (100) is being worn, extends through an opening (144) in the receptacle (114).

33. Item of clothing as claimed in any one of claims 29 to 32, **characterised in that** the body function sensor (124) comprises at least one body contact element (134) which, when the item of clothing (100) is being worn, is located in direct contact with the body, in particular the skin (146), of the wearer of the item of clothing (100).

34. Item of clothing as claimed in any one of claims 29 to 33, **characterised in that** the body function sensor (124) comprises a device for storing body function data and/or a device for wirelessly transmitting body function data to a body function data receiver.

35. Item of clothing as claimed in any one of claims 1 to 34, **characterised in that** the item of clothing (100) comprises a chest region which, when the item of clothing (100) is being worn, covers the wearer's chest.

36. Item of clothing as claimed in any one of claims 1 to 35, **characterised in that** the item of clothing (100) comprises arm parts (110).

## Revendications

1. Vêtement, en particulier chemise, comprenant un corps de base (102) et un réceptacle (114) disposé sur le corps de base (102) pour un boîtier (128) d'un capteur de fonction corporelle (124), le réceptacle (114) étant conçu comme une poche de réception (116),
**caractérisé**
**en ce que** le réceptacle (114) est disposé sur le côté intérieur du corps de base (102), et
**en ce que** la poche de réception (116) est conçue essentiellement en forme de tuyau.

2. Vêtement selon la revendication 1, **caractérisé en ce que** le réceptacle (114) est disposé sur le corps de base (102) de telle manière qu'il ne soit pas visible à l'état porté du vêtement (100).

3. Vêtement selon l'une des revendications 1 ou 2, **caractérisé en ce que** la poche de réception (116) comprend une matière textile.

4. Vêtement selon l'une des revendications 1 à 3, **caractérisé en ce que** la poche de réception (116) comprend un tricot ou un tulle.

5. Vêtement selon l'une des revendications 1 à 4, **caractérisé en ce que** la poche de réception (116) est conçue essentiellement sans couture.

6. Vêtement selon l'une des revendications 1 à 5, **caractérisé en ce que** la poche de réception (116) est reliée de manière fixe au corps de base (102) du vêtement (100).

7. Vêtement selon la revendication 6, **caractérisé en ce que** la poche de réception (116) est reliée au corps de base (102) du vêtement (100) par tricotage, couture, collage et / ou soudage.

8. Vêtement selon l'une des revendications 6 ou 7, **caractérisé en ce que** la poche de réception (116) est reliée à un col (104) du corps de base (102) du vêtement (100).

9. Vêtement selon l'une des revendications 1 à 8, **caractérisé en ce que** la poche de réception (116) comprend une matière à partir de laquelle le corps de base (102) du vêtement (100) est aussi au moins en partie formé.

10. Vêtement selon l'une des revendications 1 à 9, **caractérisé en ce que** la poche de réception (116) comprend une matière élastique, de préférence de l'élastanne.

11. Vêtement selon l'une des revendications 1 à 10, **caractérisé en ce que** la poche de réception (116) est conçue de manière souple.

12. Vêtement selon l'une des revendications 1 à 11, **caractérisé en ce que** le réceptacle (114) présente une ouverture d'accès (120) par laquelle le boîtier (128) du capteur de fonction corporelle (124) est inséré dans le réceptacle (114) et peut être retiré de celui-ci.

13. Vêtement selon la revendication 12, **caractérisé en ce que** l'ouverture d'accès (120) présente un bord flexible (122).

14. Vêtement selon la revendication 13, **caractérisé en ce que** l'ouverture d'accès (120) présente un bord (122) assuré contre un élargissement non voulu de l'ouverture d'accès (120).

15. Vêtement selon la revendication 14, **caractérisé en ce que** le bord (122) de l'ouverture d'accès (120) est assuré contre un élargissement non voulu de l'ouverture d'accès (120) par couture, soudage et / ou collage.

16. Vêtement selon l'une des revendications 12 à 15, **caractérisé en ce que** l'ouverture d'accès (120) est disposée dans une région supérieure du bord du réceptacle (114).

17. Vêtement selon l'une des revendications 1 à 16, **caractérisé en ce que** le réceptacle (114) comprend une section de bord (118) qui présente une forme adaptée à une section de bord (126) du capteur de fonction corporelle (124).

18. Vêtement selon l'une des revendications 1 à 17, **caractérisé en ce que** le réceptacle (114) présente au moins une ouverture de passage (144) pour le passage d'un élément de contact électrique (140) à travers une paroi périphérique (142) du réceptacle (114).

19. Vêtement selon la revendication 18, **caractérisé en ce que** ladite au moins une ouverture de passage (144) est disposée dans une paroi périphérique (142) du réceptacle (114) orientée, à l'état porté du vêtement (100), vers la peau (146) du porteur du vêtement (100).

20. Vêtement selon l'une des revendications 18 ou 19, **caractérisé en ce que** la paroi périphérique (142) du réceptacle (114) dans laquelle ladite au moins une ouverture de passage (114) est disposée est munie d'un revêtement.

21. Vêtement selon la revendication 20, **caractérisé en ce que** le revêtement comprend un laminé.

22. Vêtement selon l'une des revendications 20 ou 21, **caractérisé en ce que** le revêtement comprend une matière polyuréthanne.

23. Vêtement selon l'une des revendications 20 à 22, **caractérisé en ce que** le revêtement est relié à la paroi périphérique (142) du réceptacle (114) par application d'une pression plus élevée et/ou d'une température plus élevée.

24. Vêtement selon l'une des revendications 1 à 23, **caractérisé en ce que** le corps de base (102) comprend une matière textile.

25. Vêtement selon l'une des revendications 1 à 24, **caractérisé en ce que** le corps de base (102) comprend un tricot ou un tulle.

26. Vêtement selon l'une des revendications 1 à 25, **caractérisé en ce que** le corps de base (102) est conçu essentiellement sans couture.

27. Vêtement selon l'une des revendications 1 à 26, **caractérisé en ce que** le corps de base (102) comprend une matière élastique, de préférence de l'élastanne.

28. Vêtement selon l'une des revendications 1 à 27, **caractérisé en ce que** le réceptacle (114) est disposé sur le vêtement (100) de telle manière que le boîtier (128) du capteur de fonction corporelle (124) reçu dans le réceptacle (114) se trouve, à l'état porté du vêtement (100), dans la région de la poitrine du porteur.

29. Vêtement selon l'une des revendications 1 à 28, **caractérisé en ce que** le vêtement (100) comprend au moins un capteur de fonction corporelle (124) dont le boîtier (128) est disposé dans le réceptacle (114).

30. Vêtement selon la revendication 29, **caractérisé en ce que** le boîtier (128) du capteur de fonction corporelle (124) peut être retiré du réceptacle (114) sans détérioration.

31. Vêtement selon l'une des revendications 29 ou 30, **caractérisé en ce que** le capteur de fonction corporelle (124) est conçu comme un capteur de fréquence cardiaque.

32. Vêtement selon l'une des revendications 29 à 31, **caractérisé en ce que** le capteur de fonction corporelle (124) comprend au moins un élément de contact électrique (140) qui s'étend, à l'état porté du vêtement (100), à travers une ouverture de passage (144) du réceptacle (114).

33. Vêtement selon l'une des revendications 29 à 32, **caractérisé en ce que** le capteur de fonction corporelle (124) comprend au moins un élément de contact corporel (134) qui, à l'état porté du vêtement (100), se trouve en contact direct avec le corps, en particulier avec la peau (146), du porteur du vêtement (100).

34. Vêtement selon l'une des revendications 29 à 33, **caractérisé en ce que** le capteur de fonction corporelle (124) comprend un dispositif pour stocker des données de fonction corporelle et / ou un dispositif pour transmettre sans fil des données de fonction corporelle à un récepteur de données de fonction corporelle.

35. Vêtement selon l'une des revendications 1 à 34, **caractérisé en ce que** le vêtement (100) comprend une région de poitrine qui recouvre, à l'état porté du vêtement (100), la poitrine du porteur.

36. Vêtement selon l'une des revendications 1 à 35, **caractérisé en ce que** le vêtement (100) comprend des manches (110).
